# NEW EUROPEAN PATENT SPECIFICATION

(11) **EP 2 547 443 B2**
(45) Date of publication and mention of the opposition decision: **30.10.2024**
(45) Mention of the grant of the patent: 06.04.2022
(21) Application number: 11708482.2
(22) Date of filing: 17.03.2011
(51) Int. Cl.: B01J 21/04, B01J 23/66, B01J 37/02, B01J 35/00

(54) **HYDROGENATION CATALYST**
HYDRIERUNGSKATALYSATOR
CATALYSEUR D'HYDROGÉNATION

(30) Priority: 19.03.2010 EP 10157055
(43) Date of publication of application: 23.01.2013
(73) Proprietor: Shell Internationale Research Maatschappij B.V., 2596 HR The Hague (NL)
(72) Inventor: HERZFELD, Tobias, 06114 Halle (Saale) (DE); HOHEISEL, Klaus, 06237 Leuna (DE); KLEMT, Andreas, 06237 Leuna (DE); PACHULSKI, Axel, 06237 Leuna (DE); PREISING, Henri, 06108 Halle (Saale) (DE); SCHÖDEL, Rainer, 06237 Leuna (DE); VAN DER ZEIJDEN, Dolf, 06237 Leuna (DE)
(74) Representative: Shell Legal Services IP
(86) International application number: PCT/EP2011/054011
(87) International publication number: WO 2011/113881

(56) References cited:
- WO-A1-01/19763
- WO-A2-2008/127406
- DE-A1- 3 543 640
- GB-A- 686 574
- US-A- 3 243 387
- US-A- 5 889 187
- US-A1- 2005 137 433
- US-A1- 2007 027 030
- SHENG-YI LEE ET AL: "The Distribution of Active ingredients in Supported Catalysts Prepared by Impregnation", CATALYSIS REVIEWS: SCIENCE AND ENGINEERING, MARCEL DEKKER INC. NEW YORK, US, vol. 27, no. 2, 1 January 1985 (1985-01-01), pages 207 - 340, XP008097790, ISSN: 0161-4940, DOI: 10.1080/01614948508064737

## Description

The present invention relates to a method for producing supported metal catalysts, catalysts obtainable thereby and their use in a hydrogenation process.

In refineries and petrochemical facilities large amounts of hydrocarbons are produced and stored which comprise significant amounts of unsaturated hydrocarbons causing problems during the further processing steps and its storage. Such unsaturated compounds are for instance acetylene, propine, propadiene, butadienes, vinylacetylene, butines, phenylacetylene and styrene.

Acetylene is known to reduce the catalyst activity in polymerisation processes and the quality of the polymers is deteriorated. Thus, in the synthesis of polyethylene from ethylene the concentration of acetylene should be minimized.

The undesired, unsaturated compounds are removed mainly by selective hydrogenation wherein these compounds are hydrogenated, preferably to a content of less than a few ppm. It is important for the efficiency of the selective acetylene hydrogenation that the selective hydrogenation of ethylene to ethane and the oligomerization to higher hydrocarbons and the production of coke are avoided.

In the art, for the selective hydrogenation nickel sulfide, W/NiS or copper containing catalysts were used. Due to their low activity at high temperatures the formation of polymers was increased. It is also known to use supported Pd-catalysts based on aluminium oxides and silicium oxides for selective hydrogenation processes.

JP 580 178 35 discloses a Pd-Al₂O₃ catalyst with a surface area of 55 to 120 m²/g. JP 650 178 13 discloses that the crystallite size of Pd in a Pd-Al₂O₃ catalyst should be at least 5.0 nm. US 4,762,956 discloses that the selectivity of a Pd-Al₂O₃ catalyst is improved by using a mixture of three different aluminium oxide hydrates as support. AS 1 171 901 discloses a Pd-Al₂O₃ catalyst having a Pd content of 0.01 to 0.09 % by weight and a maximum pore volume of 0.4 cm³/g. GB 1,133,253 discloses a Pd-SiO₂ catalyst with a content of Pd of 0.01 to 1.0 % by weight and a support of silicic acid with a surface area of 250 to 450 m²/g. DE 697 20 235 discloses a Pd-Al₂O₃ catalyst for the selective hydrogenation of acetylene, wherein the crystallite of Pd have mainly (100) and (111) areas and wherein the adsorbed hydrogen is desorbed in two different temperature areas.

JP 54157507 discloses a Pd-Al₂O₃ catalyst whose α-Al₂O₃ support was coated with a layer of Al₂O₃ and, subsequently, calcined at temperatures of 400 to 700° C. The advantage of the Pd-Al₂O₃ catalysts and the Pd-SiO₂ catalysts used in technical application was significant in comparison with the nickel sulfide catalysts, but their durability was not satisfactory. Therefore, efforts were undertaken to modify the properties of the catalysts and the process conditions. US 4,329,530 discloses a calcium aluminate supported Pd catalyst with a Pd penetration depth of less than 300 µm. FR 2 603 578 discloses a Pd-Al₂O₃ catalyst with gold as promoting metal. US 2,802,889 discloses a method for the selective hydrogenation of acetylene with Pd catalysts comprising copper, silver, and gold wherein the content of the elements of the eighth subgroup in the metallic phases is 1 to 40 %. These catalysts are produced by impregnating a kieselguhr support with a solution of Pd/Cu, Pd-Ag, and Pd/Au salts, by drying, calcining and reducing with hydrogen at a temperature of below 450°C. DE 197 57 990 discloses supported Pd catalysts comprising Ni, Co, or Ag and Na, K, or Ca, and a layer of precipitated organic silanes. US 3,243,387 discloses a Pd-Ag-catalyst on a Fe₂O₃-αAl₂O₃ support produced by impregnating the support with a solution of metal nitrates and by subsequent drying, calcining and reduction steps.

US 4,484,015 discloses a Pd-Ag-αAl₂O₃ catalyst wherein Ag is homogenously distributed over the total catalyst and 90 % of the Pd is distributed in the outer shell region with a penetration depth of 300 µm. Furthermore, the weight ratio of Ag to Pd is from 2 to 6. The metal ions are reduced with gas containing hydrogen after impregnating, drying, and calcining steps. DE 198 40 373 discloses catalysts comprises at least one metal of the eighth subgroup with an eggshell distribution and at least one metal of the first subgroup with a homogeneous distribution over the entire catalyst. The weight ratio of the metal of the first subgroup to the metal of the eighth subgroup is not more than 1.95. US 5,510,550 discloses Pd-Ag-catalysts reduced with hydrazine, formic acid, formaldehyde or alkaliboronhydrides in an aqueous solution in the presence of alkali compounds after their impregnation, drying, and calcining. EP 0 722 776 discloses Pd-Ag-Al₂O₃ catalysts comprising KF for gas streams containing sulfur. US 2004/0248732 discloses Pd-Ag-Al₂O₃ catalysts comprising compounds selected from the group consisting of KSbF₆, KPF₆, KBF₄, and KAlF₄. KR 2000-0059743 discloses the promotion of Pd with titan oxide, whereby the selectivity of the Pd shall be increased by the SMSI effect. US 7,453,017 discloses a method wherein the SMSI effect is induced at already 300° C. In US 6,797,669 the durability of the catalysts is increased by a homogenous distribution of Pd and a metal of the first subgroup having a penetration depth of more than 300 µm, a molar ratio of Pd to the metal of the first subgroup of 1 to 20, a crystallite size of alloys of 2 to 10 nm and an average pore diameter of 40 to 100 nm. The catalysts are produced by simultaneous impregnation of the metal salts at pH values of 1 to 4, by drying, by calcining and by reducing with hydrogen. US 2006/0107424 discloses that metal aluminates of Zn, Mg and Ca can be used as supports for Pd, Ag, and alkali compositions. US 6,255,548 discloses supported Pd catalysts promoted with Ge, Sn, Pb, Re, Ga, In, Au, Ag, and Tl. The catalysts are produced using at least one water soluble metal organic composition of the promoters.

In their Examples preparing various catalysts, WO200119763 discloses the use of Zn(NO₃)₂; US2005137433 discloses the use of KF and NH₄F; US2007027030 discloses the use of NH₄Cl; and DE3543640 discloses the use of NaOH. None of these compounds is capable of reducing a transition metal.

Further, in Examples 1 and 5 of GB686574, formaldehyde was used in preparing a catalyst which is a compound which is capable of reducing a transition metal. The formaldehyde that should reduce such transition metal, was in each case applied after the transition metal was applied.

Still further, in Example 5 of US5889187, citric acid was used in preparing a catalyst which is a compound which is capable of reducing a transition metal. Reduction took place in an aqueous solution containing a catalyst and the citric acid, into which solution silver acetate was introduced.

According to the state of the art catalysts exhibit an unsatisfactory durability and selectivity, in particular when employed in a hydrogenation process, in particular in selective hydrogenation processes for hydrogenating acetylene, propine, propadiene, butadienes, vinylacetylene, butines, phenylacetylene and styrene in gaseous phase.

Therefore, the technical problem underlying the present invention is to overcome the above-identified disadvantages, in particular to provide a method for producing a catalyst and a catalyst obtained thereby for the hydrogenation of a hydrocarbon feed. Preferably the catalyst shall be suitable for the selective hydrogenation of unsaturated hydrocarbons in a hydrocarbon feed, particularly in a gaseous hydrocarbon feed, comprising unsaturated hydrocarbons, in particular acetylene, propine, propadiene, butadienes, vinylacetylene, butines, phenylacetylene and styrene, which shows a high, in particular improved, durability, selectivity or both.

Furthermore, the technical problem underlying the present invention is to provide a process for the hydrogenation of a hydrocarbon feed, in particular for the selective hydrogenation of unsaturated hydrocarbons in a hydrocarbon feed, particularly in a gaseous hydrocarbon feed, comprising unsaturated hydrocarbons, in particular acetylene, propine, propadiene, butadienes, vinylacetylene, butines, phenylacetylene and styrene, which overcomes the above identified disadvantages and in particular which is characterized by a high, in particular improved durability, selectivity or both.

The technical problem of the present invention is solved according to the teaching of the independent claims.

In particular, the present invention solves its technical problem by a method for producing a supported metal catalyst, comprising the following steps:
a) providing a catalyst support,
b) applying a first liquid medium comprising at least one_reducing agent selected from the group consisting of hydrazine, formic acid, and formaldehyde to the catalyst support provided in step a), wherein 10 to 70 %, of the total pore volume of the catalyst support provided in step a) is filled with the first liquid medium,
c) instantly after step (b),impregnating the catalyst support obtained in step b) with a second liquid medium comprising two transition metals selected from the group consisting of Pd, Pt, Cu, Ni, Au and Ag to obtain an impregnated precursor, wherein at least 91 % of the total pore volume of the catalyst support provided in step a) is filled with the first and second liquid medium,
d) drying the impregnated precursor obtained in step c) at a temperature from 80 to 350 °C to obtain a dried, impregnated precursor and
e) treating the dried, impregnated precursor obtained in step d) at a temperature from 350 to 700 °C to obtain a supported metal catalyst wherein the total pore volume of the catalyst support is determined by Hg porosimetry; and wherein the reducing agent is a compound that is capable of reducing the transition metal from the second liquid medium that is used in step c).

In the context of the present invention a "liquid medium" is preferably a solution. In the context of the present invention a "liquid medium" may also be a suspension. In a preferred embodiment of the present invention the solution or suspension is an aqueous solution or suspension.

In the context of the present invention the term "homogeneously filled" is understood that in every single pore of the catalyst support essentially the same or the same amount of a liquid medium is present after the applying and/or the impregnation. By filling the pores of the catalyst support homogenously essentially the same or the same molar content of a reducing agent and/or transition metals is present in every single pore. Thus, essentially the same or the same ratio of the reducing agent to the transition metals in every single pore of the catalyst support can be achieved.

Thus, preferably all accessible surfaces areas and volumetric regions of at least one, preferably all species, for instance micropores or macropores, of the pore system are moistened or covered homogenously.

In the context of the present invention the expression "reducing agent" means a compound in a reduction-oxidation reaction that donates an electron to another species, which meaning is common general knowledge. That is to say, in the context of the present invention, the reducing agent is a compound that is capable of reducing the transition metal from the second liquid medium that is used in step c) of the present catalyst production method. For example, where the transition metal is palladium, Pd²⁺ from said liquid medium may be reduced to Pd° (the metal) by the reducing agent (donation of 2 electrons).

In the context of the present invention the expression "and/or" used in between two elements is meant to designate that both elements linked by said term are referred to in a cumulative or alternative manner. Thus, the expression "A and/or B" encompasses the meanings "A or B" and "A and B", that means "any one of A, B or both".

"% by weight" values given in the present teaching refer, if not otherwise stated, to the weight of the total dry catalyst. In the context of the present invention, the components of the catalyst are to be selected in an overall amount to add up to 100 % by weight, most preferably not to exceed 100 % by weight.

In the context of the present invention, the term "outer shell region" is the peripheral region of the catalyst, which is defined by the presence of preferably at least 90 % of the total amount of the two transition metals impregnated in step c), preferably the silver, the palladium or both (calculated on the basis of the total catalyst weight) and which is the outer peripheral region of the catalyst, preferably a region between the surface of the catalyst and its centre having a maximum depth calculated along an axis in the diffusion direction of impregnating metal ions towards the catalyst interior, in particular along an axis drawn perpendicularly to the tangent of a given point of the surface of the catalyst towards the interior of the catalyst, of 200 µm and most preferably 150 µm. Thus, the peripheral region of the catalyst is the region, wherein preferably at least 90 % of the total amount of the at two transition metals, preferably the silver, the palladium or both contained in the total catalyst is present and which region extends from the surface of the catalyst to not more than 200 µm and most preferably 150 µm beneath the geometrical surface of the catalyst. Thus, the depth of the outer shell region is the cross-sectional depth of the outer shell region and therefore equals the distance between the geometrical surface of the catalyst and the circumference of the catalyst centre.

In the context of the present invention, the centre of the catalyst is the remainder of the catalyst, i.e. the interior region of the catalyst.

In a preferred embodiment of the present invention the diameter of the centre of the catalyst is at least 2, 3, 4, 5, 6, 7, 8, 9, 10, 11, 12, 13, 14, 15 or 20 times greater than the depth of the outer shell region.

In a preferred embodiment of the present invention the radius of the centre of the catalyst is at least 2, 3, 4, 5, 6, 7, 8, 9, 10, 11, 12, 13, 14, 15 or 20 times greater than the depth of the outer shell region. In the context of the present invention, the term "catalyst support" is understood as being a pure carrier containing no catalytically active metal or as a carrier which comprises at least one catalytically active metal, preferably transition metal, for instance palladium. Said catalytically active metal being preferably already present in the catalyst support is distributed homogeneously or inhomogeneously over the entire catalyst support.

Thus, the method of the present invention uses in step a) as a catalyst support either a carrier containing no catalytically active metal or in another embodiment a carrier which already contains at least one catalytically active metal, preferably transition metal, and which is, subsequent to process step b), in step c), impregnated with two transition metals.

In the context of the present invention the term "hydrocarbons" refers both to hydrocarbons consisting solely of C- and H-atoms and to hydrocarbons additionally comprising at least one functional group and/or at least one heteroatom, for instance nitroaromatics.

In a particularly preferred embodiment the term "hydrocarbons" therefore refers to hydrocarbons consisting solely of C- and H-atoms. In a furthermore preferred embodiment the term "hydrocarbons" refers to hydrocarbons additionally comprising at least one functional group and/or at least one heteroatom, for instance nitroaromatics.

In the context of the present invention, the term "inhomogeneous" means that a metal is unevenly distributed in the entire catalyst. The inhomogeneous distribution is preferably obtained by impregnation of the support with the catalytically active metal, in particular Ag or Pd.

Other methods to obtain an inhomogeneous distribution may be used and are known in the art. Accordingly, by an appropriate choice of the support material, the metals and/or metal precursors used and the process conditions, in particular the pH, the temperature and the pressure, an inhomogeneous distribution may also be achieved, even if the liquid medium is homogeneously distributed in the entire catalyst. Thus, in one embodiment an inhomogeneous distribution of a metal may also be achieved by a homogeneous distribution of the liquid media used in the present invention. Further methods, such as precipitation or dropping methods, may be used to achieve the desired inhomogeneous distribution.

In another embodiment a homogeneous distribution of the metal may be achieved by impregnation, precipitation or other methods by applying appropriate process conditions.

In the context of the present invention the term "selective hydrogenation of a hydrocarbon feed" is meant to designate a process according to which a hydrocarbon feed containing two groups of unsaturated hydrocarbons is subjected to a hydrogenation, wherein a first group of specific unsaturated hydrocarbons, in particular highly unsaturated hydrocarbons such as alkynes and di- or oligounsaturated hydrocarbons such as alkadienes, alkatrienes or alkapolyenes, in particular acetylene, butadiene, propine and propadiene, is hydrogenated and wherein a second group of other less unsaturated hydrocarbons, in particular monounsaturated hydrocarbons, namely alkenes, such as ethylene, remains substantially in the unsaturated form. Thus, a selective hydrogenation according to the present invention is preferably a hydrogenation wherein a first group of specific unsaturated hydrocarbons, in particular comprising acetylene, is hydrogenated, preferably to ethylene, and a second group of other specific unsaturated hydrocarbons, in particular comprising ethylene, is not hydrogenated and remains monounsaturated.

In the context of the present invention the CO-chemisorption is determined by an impulse chemisorption method using AutoChem 2920 (company: Micromeritics) at a chemisorption temperature of 0 °C. For the analysis the catalysts are activated at 200 °C (P. A. Webb, C. Orr, Analytical Methods in Fine Particle Technology, Micromeritics Instrument Corporation, 1997).

In the context of the present invention the composition of the metal crystallites is determined by the following method: The outer shell region of the catalyst in a depth of 100 µm (calculated from the surface) was abrased and isolated with a microtome. The obtained material was added into ethanol and nebulised by means of ultrasound. Subsequently, the nebulised material was spread onto a carrier film. For the energy dispersive microanalysis a scanning transmission electron microscope JEM 2000 ex (Jeol, Japan), an EDX spectrometer Genesis (EDAX, USA) and a digital image processing system DISS 5 (point electronic, Germany) were used. The selective analyses were carried out at an excitation voltage of 200 kV. During the measurements the electron beam was moved geometrically along the crystallites. For the quantitative analysis of the obtained spectra the K-α lines, for instance of Pd and/or Ag, were used and the quantitative relation of the at least two masses was determined.

The pore volume, in particular the total and partial pore volume, is determined by Hg porosimetry (AutoPor IV; company: Micromeritics; P. A. Webb, C. Orr, Analytical Methods in Fine Particle Technology, Micromeritics Instrument Corporation, 1997).

Thus, the technical problem underlying the present invention is solved by the production of a supported metal catalyst, wherein in a first step a catalyst support is provided. In a second step a first liquid medium, preferably a first solution or suspension, is applied to the catalyst support. The first liquid medium comprises at least one, preferably one, reducing agent. In the second step 10 to 70 %, of the total pore volume of the catalyst support is filled, especially homogeneously, with the first liquid medium, comprising the at least one reducing agent. In a third step the catalyst support filled with the first liquid medium, comprising the at least one reducing agent, is impregnated with a second liquid medium, preferably a second solution or suspension, comprising two transition metals. After the impregnation with the second liquid medium at least 91 % of the total pore volume of the catalyst support is filled, preferably homogeneously, for instance at least 91 % of the total pore volume is filled with the first and second liquid medium, preferably with the first and second solution or suspension. In a fourth step the precursor impregnated with the first and the second liquid medium is dried at a temperature of 80 to 350 °C. In a fifth step the dried, impregnated precursor is treated, especially calcined, at a temperature from 350 to 700 °C to obtain the desired supported metal catalyst.

Presently it could be shown that by any of the methods according to the present invention a very strong interaction between one metal, preferably palladium, and a second metal, in particular Ag, optionally also to a third metal can be obtained. Said strong interaction allows the formation to an alloy and influences the electronic behaviour of the metals.

In the present invention the at least one reducing agent of step b) is selected from the group consisting of hydrazine, formic acid and formaldehyde.

In a preferred embodiment of the present invention the at least one reducing agent is hydrazine.

In a preferred embodiment of the present invention 20 to 80 %, in particular 30 to 70 % and in particular 35 to 70 % of the total pore volume of the catalyst is filled with the first liquid medium.

In a preferred embodiment of the present invention 20 to 80 %, in particular 30 to 70 % and in particular 30 to 65 % of the total pore volume of the catalyst is filled with the second liquid medium.

In a preferred embodiment of the present invention the first and second liquid medium is filled into the pores of the catalyst to add up to at least 91 %, preferably at least 92 %, 93 %, 94 %, 95 %, 96 %, 97 %, 98 %, 99 % and in particular 100 % of the total pore volume.

In a preferred embodiment of the present invention at least 91 %, preferably at least 92 %, 93 %, 94 %, 95 %, 96 %, 97 %, 98 %, 99 % and in particular 100 % of the total pore volume is filled with the first and second liquid medium.

In another preferred embodiment of the present invention the first and the second liquid medium is applied to the pores of the catalyst in an amount to add up to more than 100 % of the total pore volume, preferably 120 % at the maximum, preferably 110 % at the maximum and in particular 105 % at the maximum.

In a preferred embodiment of the present invention the pores of the catalyst support are filled with the first and the second liquid medium such as to locate the concentration maximum of the two transition metals in the outer shell region, namely in a subregion thereof within a depth from 50 to 200 µm, preferably 50 to 150 µm, preferably 70 to 130 µm, preferably 90 to 110 µm, calculated from the surface of the catalyst towards its centre.

Thus, the invention foresees in a preferred embodiment that within the outer shell region as defined above, that means within the peripheral region of the catalyst where preferably at least 90 % of the total amount of the two transition metals is located and which is defined by a maximal depth of 200 µm beneath the geometrical surface, that means counted from the surface of the catalyst, a subregion is present, in which a concentration maximum of the two transition metals is present and wherein said subregion is located in a region from a depth from 50 µm counted from the surface of the catalyst to 200 µm counted from the surface of the catalyst. Thus, the maximum of the two transition metal concentration is preferably contained in a layer in a specified region of the outer shell region.

In the present invention the second liquid medium comprises two transition metals.

In the present invention the two transition metals are selected from the group consisting of Pd, Pt, Cu, Ni, Au and Ag.

In a preferred embodiment of the present invention one of the transition metals is Pd and the one other transition metal is selected from the group consisting of Pt, Cu, Ni, Au and Ag.

In a preferred embodiment of the present invention one transition metal is Pt and the one other transition metal is selected from the group consisting of Pd, Cu, Ni, Au and Ag.

In a preferred embodiment of the present invention the stoichiometric excess of the reducing agent to the two transition metals is from 2 to 50, preferably 5 to 10, preferably 6 to 9 and in particular 7 to 8.

In a preferred embodiment of the present invention the liquid medium is applied in step b) by spraying the liquid medium onto the catalyst. In a preferred embodiment of the present invention, the liquid medium in step c) is impregnated by spraying the liquid medium onto the catalyst support. Preferably, both step b) and c) are carried out in form of spraying the liquid mediums. By spraying the liquid mediums onto the catalyst support a particular homogeneous distribution is obtained.

In a preferred embodiment of the present invention the spraying of the liquid medium onto the catalyst is carried out with a nozzle capable of finely dispersing liquids such as solutions or suspensions.

In a preferred embodiment of the present invention the first liquid medium is homogeneously distributed in the pores of the catalyst support. In a preferred embodiment of the present invention the second liquid medium is homogeneously distributed in the pores of the catalyst support.

In a preferred embodiment of the present invention the first and the second liquid mediums are homogeneously distributed in the pores of the catalyst support.

In the present invention step c) is carried out instantly after step b).

In the present invention the drying temperature in step d) is from 80 to 350 °C, preferably 90 to 300 °C, preferably 100 to 250 °C, preferably 110 to 200 °C and in particular 120 to 150 °C.

In a preferred embodiment of the present invention the drying of the impregnated precursor in step d) is carried out to a dry residue of 99 %, preferably 99.5 %, preferably 99.9 % and in particular 100 %.

In a preferred embodiment of the present invention the drying can be carried out under static or dynamic conditions, for instance in a fixed bed, or in a moving bed.

In a preferred embodiment of the present invention the catalyst according to the present invention is inerted, preferably at a temperature from 300 to 350°C, preferably 350°C, preferably after the drying and preferably before the treating, preferably the calcining.

In a preferred embodiment of the present invention the inerting is carried out at the same temperature as the drying.

In a preferred embodiment of the present invention the dried impregnated precursor obtained in step d) is in a further step d') flushed with hydrogen, preferably before the treating, in particular the calcining, in step e).

In a preferred embodiment of the present invention the flushing with hydrogen carried out in step d') is performed for 2 to 20 minutes, preferably 2 to 5 minutes.

In a preferred embodiment the present invention comprises both the inerting and the flushing step.

A preferred embodiment foresees that subsequently to the drying step d) the dried, impregnated catalyst is inerted, subsequently flushed with hydrogen and then treated, preferably calcined, in step e).

In a preferred embodiment of the present invention the treating, especially the calcining, in step e) is carried out at a temperature from 400 to 700 °C, preferably 500 to 700 °C and in particular 600 to 700 °C.

In a preferred embodiment of the present invention the process for-sees to subject the catalyst to a nitrogen stream while the temperature is increased to the temperature of treating, preferably calcining.

In a preferred embodiment of the present invention the treating in step e) is carried out with a nitrogen-containing gas, preferably air or preferably nitrogen.

In a preferred embodiment of the present invention the treatment, preferably calcining, is carried out in a fix bed or in a moving bed reactor.

In a preferred embodiment of the present invention the selectivity and/or durability of the catalysts is further increased by adding before and/or during the treating in step e) to the nitrogen-containing gas, preferably to the nitrogen, used therefore a gas which is preferably selected from the group consisting of hydrogen, air, oxygen, water vapour and NOₓ, with a content from 0 to 500 ppmv (parts per million per volume), preferably 10 to 400 ppmv, preferably 20 to 300 ppmv, preferably 30 to 200 ppmv and in particular 50 to 100 ppmv.

In a preferred embodiment of the present invention the dried and impregnated precursor is treated, preferably is calcined, in step e) for 1 to 20 hours, preferably 2 to 18 hours, preferably 3 to 15 hours, preferably 5 to 10 hours and in particular 5 to 8 hours.

In a preferred embodiment of the present invention the dried and impregnated precursor is treated, preferably is calcined, in step e) at a GHSV (gas hourly space velocity) from 10 to 5000 v/vh, preferably 1000 to 4500 v/vh and in particular 3000 to 4000 v/vh.

In a preferred embodiment of the present invention the catalyst is cooled after the treating, preferably calcining, in step e) in a nitrogen containing gas, for instance under nitrogen protective atmosphere, preferably to a temperature below 50 °C.

The technical problem underlying the present invention is also solved by a catalyst of the present invention, obtained by any one of the methods according to the present invention.

In a preferred embodiment a catalyst, obtained by any one of the methods, is provided comprising two transition metals and a support, which catalyst comprises an outer shell region having a maximum depth of 200 µm or 150 µm and a centre, wherein preferably at least 90 % of the total amount of the two transition metals is distributed in the outer shell region.

The present invention solves its underlying technical problem also by the provision of a metal catalyst obtained by the above-identified method, particularly a method for producing a supported metal catalyst according to the present invention. A catalyst of the present invention is a catalyst comprising two metals, that means a two-metal catalyst which comprises two transition metals, preferably silver and palladium, and a support, which catalyst comprises an outer shell region having a maximum depth of 200 µm or 150 µm and a centre, wherein preferably at least 90 % of the total amount of the two transition metals, in particular silver and palladium, (calculated on the weight of total catalyst) are distributed in the outer shell region and wherein at least 70 %, preferably at least 80 %, in particular at least 90 % of the total amount of metal crystallites contained in the catalyst comprise the two transition metals, in particular silver and palladium. In a particularly preferred embodiment a catalyst according to the above is provided, wherein the concentration maximum of the two transition metals, preferably silver and palladium, is located in the outer shell region in subregion thereof which is located in a layer in a depth from 50 to 200 µm of the catalyst. The catalyst prepared by the present invention surprisingly shows a very high catalyst lifetime, that means durability, and a particular high selectivity for the unsaturated hydrocarbons to be removed.

In a preferred embodiment of the present invention the two transition metals, preferably palladium and/or silver, are distributed inhomogeneously in the overall catalyst, preferably is located in the outer shell region and in particular solely in the outer shell region.

In a preferred embodiment the present invention provides a Pd-Ag-catalyst comprising palladium and silver and a support, which catalyst comprises an outer shell region having a maximum depth of 200 µm or 150 µm and a centre, wherein at least 90 % of the total amount of the silver and the palladium (calculated on the weight of the total catalyst) are distributed in the outer shell region and wherein at least 70 %, preferably at least 80 %, in particular at least 90 % of the total amount of the metal crystallites contained in the catalyst comprise silver and palladium.

In a preferred embodiment of the present invention at least 90 %, preferably at least 91 %, 92 %, 93 %, 94 %, 95 %, 96 %, 97 %, 98 %, 99 % and most preferably 100 % of the total amount of the two transition metals (calculated on the basis of the total catalyst) is distributed in the outer shell region.

In a preferred embodiment of the present invention at least 90 %, preferably at least 91 %, 92 %, 93 %, 94 %, 95 %, 96 %, 97 %, 98 %, 99 % and in particular 100 % of the total amount of the silver (calculated on the basis of the total catalyst) is distributed in the outer shell region.

In a preferred embodiment of the present invention at least 90 %, preferably at least 91 %, 92 %, 93 %, 94 %, 95 %, 96 %, 97 %, 98 %, 99 % and in particular 100 % of the total amount of the palladium (calculated on the basis of the total catalyst) is distributed in the outer shell region.

In a preferred embodiment of the present invention at least 90 %, preferably at least 91 %, 92 %, 93 %, 94 %, 95 %, 96 %, 97 %, 98 %, 99 % and in particular 100 % of the total amount of the palladium and the silver (calculated on the basis of the total catalyst) is distributed in the outer shell region.

In a preferred embodiment of the present invention at least 70 %, preferably at least 80 %, preferably at least 90 %, preferably at least 91 %, 92 %, 93 %, 94 %, 95 %, 96 %, 97 %, 98 %, 99 % and in particular 100 % of the total amount of the metal crystallites contained in the catalyst comprise the at least two transition metals.

In a preferred embodiment of the present invention at least 70 %, preferably at least 80 %, preferably at least 90 %, preferably at least 91 %, 92 %, 93 %, 94 %, 95 %, 96 %, 97 %, 98 %, 99 % and in particular 100 % of the total amount of the metal crystallites contained in the catalyst comprise both metals silver and palladium.

In a preferred embodiment of the present invention the metal crystallites contained in the catalyst consist of silver and palladium.

In a further preferred embodiment of the present invention, silver and palladium are the only metals, in particular the only catalytically active metals, in the entire catalyst.

In a preferred embodiment of the present invention the palladium concentration is from 0.01 to 2.50, preferably 0.1 to 2.0, preferably 0.5 to 1.5 and in particular 0.8 to 1.2 (based on the total weight of the catalyst).

In a preferred embodiment of the present invention the palladium concentration is from 0.02 to 0.05 % by weight, preferably 0.03 to 0.04 % by weight and in particular 0.035 % by weight (based on the total weight of the catalyst).

In a preferred embodiment of the present invention the platinum concentration is from 0.01 to 2.50, preferably 0.1 to 2.0, preferably 0.5 to 1.5 and in particular 0.8 to 1.2 (based on the total weight of the catalyst).

In a preferred embodiment of the present invention the gold concentration is from 0.01 to 2.50, preferably 0.1 to 2.0, preferably 0.5 to 1.5 and in particular 0.8 to 1.2 (based on the total weight of the catalyst).

In a preferred embodiment of the present invention the silver concentration is from 0.01 to 2.50, preferably 0.1 to 2.0, preferably 0.5 to 1.5 and in particular 0.8 to 1.2 (based on the total weight of the catalyst).

In a preferred embodiment of the present invention the silver concentration is from 0.0068 to 0.03 % by weight, preferably 0.01 to 0.03 % by weight and in particular 0.01 to 0.025 % by weight (based on the total weight of the catalyst).

In a preferred embodiment of the present invention the copper concentration is from 0.01 to 5.00, preferably 0.1 to 4.0, preferably 0.5 to 3.0 and in particular 1.0 to 2.0 (based on the total weight of the catalyst).

In a preferred embodiment of the present invention the nickel concentration is from 0.01 to 5.00, preferably 0.1 to 4.0, preferably 0.5 to 3.0 and in particular 1.0 to 2.0 (based on the total weight of the catalyst).

In a preferred embodiment of the present invention the palladium concentration is from 0.02 to 0.05 % by weight, preferably 0.03 to 0.04 % by weight and in particular 0.035 % by weight (based on the total weight of the catalyst) and the silver concentration is from 0.0068 to 0.03 % by weight, preferably 0.01 to 0.03 % by weight and in particular 0.01 to 0.025 % by weight (based on the total weight of the catalyst).

In a preferred embodiment of the present invention the weight ratio of the palladium to the silver is from 1.7 to 3.0, preferably 2.0 to 3.0 and in particular is 2.5.

In a preferred embodiment, the present invention provides a catalyst, wherein the concentration maximum of the two transition metals, preferably of the silver and the palladium, is located in the outer shell region, namely in a subregion thereof within a depth from 50 to 200 µm, preferably 50 to 150 µm, preferably 70 to 130 µm, preferably 90 to 110 µm, calculated from the surface of the catalyst towards its centre. Thus, the invention foresees in a preferred embodiment that within the outer shell region as defined above, beneath the geometrical surface, that means counted from the surface of the catalyst, a subregion is present, in which a concentration maximum of the silver and the palladium is present and wherein said subregion is located in a region from a depth from 50 µm counted from the surface of the catalyst to 200 µm counted from the surface of the catalyst. Thus, the maximum of the silver and palladium concentration is preferably contained in a layer in a specified region of the outer shell region.

In a preferred embodiment of the present invention the chemisorption of carbon monoxide is from 0.10 to 0.70 pmol CO/g of the Pd-Ag-catalyst, preferably 0.20 to 0.50 pmol CO/g, preferably 0.15 to 0.40 µmol CO/g, in particular 0.25 to 0.40 µmol CO/g catalyst.

In a preferred embodiment of the present invention the BET surface area is from 5 to 18 m²/g of the Pd-Ag-catalyst, preferably 7 to 15 m²/g and in particular 9 to 13 m²/g of the catalyst.

In a preferred embodiment of the present invention the support is selected from the group consisting of C, TiO₂, Al₂O₃, ZrO₂ and SiO₂. As catalyst support, also modifications of C, TiO₂, Al₂O₃, ZrO₂ and SiO₂ can be used. Preferably, combinations of the catalyst supports selected from the group consisting of C, TiO₂, Al₂O₃, ZrO₂ and SiO₂ can be used.

In a preferred embodiment of the present invention the catalyst support is Al₂O₃ or a modification thereof.

In a preferred embodiment of the present invention the catalyst may be in the form of hollow-cylinders, tablets, spheres or extrudates.

In a preferred embodiment of the present invention the catalyst has a diameter of 1.5 to 8.0 mm, preferably 3.7 to 8.0 mm, preferably 3.2 to 5.8 mm, preferably 3.3 to 5.7 mm, preferably 3.4 to 5.5 mm.

In a preferred embodiment of the present invention the centre of the catalyst has a diameter of 1.0 to 7.7 mm, preferably 2.2 to 7.2 mm, preferably 3.0 to 4.4 mm, preferably 3.7to 4.3 mm, preferably 3.9 to 4.1 mm, in particular 4.0 mm.

In a preferred embodiment of the present invention the ratio of the radius of the centre to the distance between the geometrical surface of the catalyst and the circumference of the catalyst centre is from 15 to 2, preferably 12 to 4, preferably 10 to 6, particularly 9 to 7 and most preferably 8.

In a preferred embodiment of the present invention the, preferably spherical, catalyst has a diameter of 1.5 to 3.0 mm, which catalyst comprises an outer shell region having a maximum depth of 200 µm or 150 µm and a centre, wherein preferably at least 90 % of the total amount of the two transition metals, in particular silver and palladium, (calculated on the weight of total catalyst) are distributed in the outer shell region. The centre of said preferred catalyst has a diameter of 1.0 to 2.7 mm and the ratio of the radius of the centre to the distance between the geometrical surface of the catalyst and the circumferences of the catalyst centre is from 2 to 15.

In a preferred embodiment of the present invention the catalyst has a diameter of 3.0 to 8.0 mm. The centre of said preferred catalyst has a diameter of 2.2 to 7.7 mm and the ratio of the radius of the centre to the distance between the geometrical surface of the catalyst and the circumferences of the catalyst centre is from 2 to 15.

The catalyst according to the present invention preferably can be used in a hydrogenation process, in particular a selective hydrogenation of a hydrogen feed comprising unsaturated hydrocarbons, with a particular long catalyst lifetime allowing significantly increased cycle times. Based on the particular high durability of said catalyst hydrogenation processes can be repeated more often, preferably in case of a batchwise operation, before the said catalyst has to be regenerated. Advantageously, the catalyst according to the present invention reduces the formation of green oil.

The technical problem of the present invention is also solved by a method for the hydrogenation of a hydrocarbon feed comprising acetylene, preferably in gaseous phase, in particular a method for the selective hydrogenation of a hydrocarbon feed containing unsaturated hydrocarbons, in particular a first group of undesired, that means highly unsaturated hydrocarbons, in particular aromatics, alkynes and/or di-, tri- or polyunsaturated hydrocarbons, particularly alkadienes, alkatrienes or alkapolyenes, such as acetylene, propine, propadiene, butadienes, vinylacetylene, butines, phenylacetylene and/or styrene and a second group of desired, that means less unsaturated hydrocarbons, in particular monounsaturated hydrocarbons, namely alkenes, particularly ethylene, wherein the hydrocarbon feed is contacted under suitable hydrogenating conditions with the catalyst according to the present invention so as to remove the undesired first group of unsaturated hydrocarbons, in particular to hydrogenate them, preferably to a desired less unsaturated hydrocarbon, thereby leaving the second group of unsaturated hydrocarbons in their monounsaturated form. Thus, the present invention provides a process for the selective hydrogenation of highly unsaturated hydrocarbons in the presence of less unsaturated hydrocarbons characterized by the use of a catalyst according to the present invention.

The catalyst according to the present invention can be used in a hydrogenation process, in particular a selective hydrogenation of a hydrogen feed comprising unsaturated hydrocarbons, with a particular long catalyst lifetime allowing significantly increased cycle times. Based on the particular high durability of said catalyst hydrogenation processes can be repeated more often, before the said catalyst has to be regenerated or in a continuous fixed bed the lifetime and/or conversion is increased. Advantageously, the catalyst according to the present invention reduces the formation of higher hydrocarbons.

Thus, in a preferred embodiment of the present invention the hydrogenation is carried out in the gas phase.

In a further preferred embodiment, the present hydrogenation process is carried out according to the conditions of a front-end or tail-end hydrogenation process, preferably for the hydrogenation of C2 to C3 hydrocarbons.

In a preferred embodiment of the present invention the hydrocarbon feed comprises in its first group of unsaturated hydrocarbons acetylene, in particular in the presence of less unsaturated hydrocarbons. In a preferred embodiment, the present process foresees to preferably reduce acetylene to ethylene, in particular in the presence of less unsaturated hydrocarbons, preferably ethylene.

In a preferred embodiment acetylene is hydrogenated selectively to ethylene.

In a preferred embodiment of the present invention the hydrocarbon feed is contacted in the hydrogenation process with the catalyst at a temperature from 10 to 250 °C, preferably 30 to 200 °C, preferably 50 to 180 °C and in particular 60 to 120 °C.

In a preferred embodiment of the present invention the hydrocarbon feed is contacted in the hydrogenation process with the catalyst at a pressure from 0.5 to 90 bars, preferably 0.5 to 60 bars, preferably 5 to 20 bars and in particular 10 to 20 bars.

In a preferred embodiment of the present invention the hydrocarbon feed is conducted in the hydrogenation process with the catalyst at a GHSV (gas hourly space velocity) from 1000 to 15000 v/vh, 3000 to 12000 v/vh, preferably 3000 to 7000 v/vh and in particular 3000 to 4000 v/vh.

In a preferred embodiment of the present invention the hydrocarbon feed is contacted in the hydrogenation process with a catalyst without the use of carbon monoxide as moderator. Furthermore, the hydrogenation can be carried out without carbon monoxide, namely is a monocarboxide-free process.

In a preferred embodiment of the present invention the molar ratio of hydrogen to the acetylene is from 0.8 to 1.8, preferably 1.0 to 1.5 and in particular 1.0 to 1.3, particularly 1.2.

In a preferred embodiment of the present invention the molar ratio of the hydrogen to the acetylene is from 1.8 to 100, preferably 1.8 to 70, preferably 1.8 to 30 and in particular 1.8 to 10.

In a preferred embodiment of the present invention poisoning metals, such as mercury and arsenic, are removed prior to the hydrogenation process, preferably using a guard bed.

In a preferred embodiment for the process of hydrogenation the hydrocarbon feed is contacted with hydrogen to obtain the hydrogenated products.

Further preferred embodiments are the subject matter of the subclaims.

Further advantages of the present invention are illustrated by way of the following examples:

### Examples

### Example 1 (According to the Present Invention)

400 g of an α-aluminium oxide support (tablets 4x4 mm) were impregnated with a solution of an aqueous hydrazine (with a 2 % content of hydrazine). The volume of the solution is equivalent to 35 % of the total pore volume of the support. Subsequently, the support was impregnated with an aqueous solution of silver nitrate and palladium nitrate. The concentrations of the noble metals in the solution were chosen in that way, that the content of palladium is 0.035 % by weight and of silver is 0.025 % by weight in the catalyst, when the solution is absorbed completely. The volume of the noble metal containing solution is equivalent to 65 % of the total pore volume of the support. The impregnated catalyst precursor was dried completely in a moving bed and, subsequently, calcined for five hours at a temperature of 630° C under a nitrogen atmosphere.

### Example 2 (According to the Present Invention)

400 g of an α-aluminium oxide support (tablets 4x4 mm) were impregnated with a solution of an aqueous hydrazine (with a 1 % content of hydrazine). The volume of the solution is equivalent to 70 % of the total pore volume of the support. Subsequently, the support was impregnated with an aqueous solution of silver nitrate and palladium nitrate. The concentrations of the noble metals in the solution were chosen in that way, that the content of palladium is 0.035 % by weight and of silver is 0.022 % by weight in the catalyst, when the solution is absorbed completely. The volume of the noble metal containing solution is equivalent to 30 % of the total pore volume of the support. The impregnated catalyst precursor was dried completely in a moving bed, inerted at a temperature of 350°C, subsequently, flushed with hydrogen for two minutes and, subsequently, calcined for five hours at a temperature of 630° C under a nitrogen atmosphere.

### Example 3 (According to DE 695 14 283)

400 g of an α-aluminium oxide support (spherical body 2 to 4 mm) were impregnated with 240 ml of a solution consisting of citric acid, palladium nitrate, and silver nitrate. Subsequently, the impregnated material was dried at a temperature of 120° C and calcined at a temperature of 750° C under air. The obtained catalyst comprises 0.05 % by weight of palladium and 0.005 % by weight of silver. The catalyst was reduced at a temperature of 120° C before the catalytic measurement.

### Analytics

The CO-chemisorption was determined by an impulse chemisorption method using AutoChem 2920 (company: Micromeritics) at a chemisorption temperature of 0 °C. For the analysis the catalysts were activated at 200 °C (P. A. Webb, C. Orr, Analytical Methods in Fine Particle Technology, Micromeritics Instrument Corporation, 1997).

The composition of the metal crystallites was determined by the following method:
The outer shell region of the catalyst in a depth of 100 µm (calculated from the surface) was abrased and isolated with a microtome. The obtained material was added into ethanol and nebulised by means of ultrasound. Subsequently, the nebulised material was spread onto a carrier film. For the energy dispersive microanalysis a scanning transmission electron microscope JEM 2000 ex (Jeol Japan), an EDX spectrometer Genesis (EDAX, USA) and a digital image processing system DISS 5 (point electronic, Germany) were used. The selective analyses were carried out at an excitation voltage of 200 kV. During the measurements the electron beam was moved geometrically along the crystallites. For the quantitative analysis of the obtained spectra the K-α lines, for instance of Pd and/or Ag, were used and the quantitative relation of the at least two masses was determined.

The pore volume, in particular the total and partial pore volume, was determined by Hg porosimetry (AutoPor IV; company: Micromeritics; P. A. Webb, C. Orr, Analytical Methods in Fine Particle Technology, Micromeritics Instrument Corporation, 1997).

### Catalytic Properties of the Catalysts

The catalytic performances of the catalysts were characterized by the following test. Experimental conditions were chosen which closely resembled those of a first reactor of a technical tail end reactor:
- 15 ml catalyst in five layers
- 85 ml dilution (α-Al₂O₃)
- GHSV 4,000 v/vh
   feed composition (Mol-%) 1.0 C₂H₂ 1.0 H₂ rest: Ar 30 C₂H₄ 1.0 C₃H₈
- pressure: 10 bar
- operation mode: 3,000 ppm acetylene at reactor outlet
- criteria: selectivity and average reactor temperature versus time on stream

The conversion of acetylene was calculated as follows: [(acetylene inlet concentratio - acetylene outlet concentration)/acetylene inlet concentration] * 100.

The selectivity towards ethylene was calculated as follows: [(ethylene outlet concentration - ethylene inlet concentration)/(acethylene inlet concentration - acethylene outlet concentration)] * 100.

From Table 1 below it is evident that the catalyst according to the present invention exhibits a high durability.

**Table 1:**

| Example | decrease of selectivity after 150 hrs. in % | increase of temperature after 150 hrs.* |
|---|---|---|
| 1 | 78.4 % - 71.4 % = 7.0 % | 55° C → 59° C |
| 2 | 77.9 % - 71.1 % = 6.8 % | 56° C → 58° C |
| 3 | 79.2 % - 23.1 % = 56.1 % | 51 °C - 68 °C |
| * The temperature being necessary for an acetylene conversion of 70 % under the conditions mentioned | | |

### Properties of the Catalysts

**Table 2:**

| | Catalyst of Example 1 (according to the present invention) | Catalyst of Example 2 (according to the present invention) | Catalyst of Example 3 (comparison) |
|---|---|---|---|
| Pd content in % by weight | 0.035 | 0.035 | 0.05 |
| Ag content in % by weight | 0.025 | 0.022 | 0.005 |
| Pd content in the outer shell region (< 400 µm) | 100 % | 100 % | 100 % |
| Content of crystallites comprising Ag and Pd | 100 % | 100 % | 60 % |
| Ag content in the outer shell region (< 400 µm) | 100 % | 100 % | 50 % |
| CO-chemisorption in µmol/g | 0.12 | 0.17 | 0.61 |

## Claims

1. A method for producing a supported metal catalyst, comprising the following steps:
a) providing a catalyst support,
b) applying a first liquid medium comprising at least one reducing agent selected from the group consisting of hydrazine, formic acid and formaldehyde to the catalyst support provided in step a), wherein 10 to 70 %, of the total pore volume of the catalyst support provided in step a) is filled with the first liquid medium,
c) instantly after step b), impregnating the catalyst support obtained in step b) with a second liquid medium comprising two transition metals selected from the group consisting of Pd, Pt, Cu, Ni, Au and Ag to obtain an impregnated precursor, wherein at least 91 % of the total pore volume of the catalyst support provided in step a) is filled with the first and the second liquid medium,
d) drying the impregnated precursor obtained in step c) at a temperature from 80°C to 350°C to obtain a dried, impregnated precursor and
e) treating the dried, impregnated precursor obtained in step d) at a temperature from 350°C to 700°C to obtain the supported metal catalyst;
wherein the total pore volume of the catalyst support is determined by Hg porosimetry; and
wherein the reducing agent is a compound that is capable of reducing the transition metal from the second liquid medium that is used in step c).

2. A two-metal catalyst obtained by the method of claim 1, comprising two transition metals and a support, which catalyst comprises an outer shell region having a maximum depth of 200 µm and a centre wherein at least 90 % of the total amount of the two transition metals are distributed in the outer shell region and wherein at least 90 % of the total amount of metal crystallites contained in the catalyst comprise the two transition metals; and wherein the two transition metals are selected from the group consisting of Pd, Pt, Cu, Ni, Au and Ag.

3. The catalyst according to claim 2, comprising silver, palladium, and a support, which catalyst comprises an outer shell region having a maximum depth of 200 µm and a centre, wherein at least 90% of the total amount of the silver and the palladium are distributed in the outer shell region and wherein at least 90% of the total amount of metal crystallites contained in the catalyst comprise silver and palladium.

4. The catalyst according to claim 3, wherein the Pd concentration is from 0.02 to 0.05 % by weight, based on the total weight of the catalyst.

5. The catalyst according to claims 3 or 4, wherein the weight ratio of the palladium to the silver is from 1.7 to 3.0.

6. A method for the hydrogenation, preferably for the selective hydrogenation, of a hydrocarbon feed comprising acetylene, preferably comprising a first and a second group of unsaturated hydrocarbons, wherein the hydrocarbon feed is contacted under suitable hydrogenating conditions with the catalyst according to any one of the claims 2 to 5 the unsaturated hydrocarbons in the hydrocarbon feed, preferably in the first group, are hydrogenated.

7. The method for the hydrogenation according to claim 6, wherein the hydrocarbon feed comprises in its first group of unsaturated hydrocarbons acetylene.

8. The method for the hydrogenation according to claim 6 or 7, wherein the hydrocarbon feed is contacted with the catalyst at a temperature from 10 to 250°C.

9. The method for the hydrogenation according to any of the claims 6 to 8, wherein the hydrocarbon feed is contacted with the catalyst at a pressure from 0.5 to 90 bars.

10. The method for the hydrogenation according to any of claims 6 to 9, wherein the hydrocarbon feed is contacted with the catalyst at a GHSV (gas hourly space velocity) from 1000 to 15000 v/vh.

11. The method of the hydrogenation according to any of claims 6 to 10, wherein the hydrocarbon feed is contacted with a stream of hydrogen.

## Patentansprüche

1. Verfahren zum Herstellen eines geträgerten Metallkatalysators, umfassend die folgenden Schritte:
a) Bereitstellen eines Katalysatorträgers,
b) Aufbringen eines ersten flüssigen Mediums, umfassend mindestens ein Reduktionsmittel ausgewählt aus der Gruppe bestehend aus Hydrazin, Ameisensäure und Formaldehyd, auf den in Schritt a) bereitgestellten Katalysatorträger, wobei 10 bis 70 % des gesamten Porenvolumens des in Schritt a) bereitgestellten Katalysatorträgers mit dem ersten flüssigen Medium gefüllt sind,
c) unmittelbar nach Schritt b) Imprägnieren des in Schritt b) erhaltenen Katalysatorträgers mit einem zweiten flüssigen Medium, umfassend zwei Übergangsmetalle ausgewählt aus der Gruppe bestehend aus Pd, Pt, Cu, Ni, Au und Ag, um einen imprägnierten Vorläufer zu erhalten, wobei mindestens 91 % des gesamten Porenvolumens des in Schritt a) bereitgestellten Katalysatorträgers mit dem ersten und dem zweiten flüssigen Medium gefüllt sind,
d) Trocknen des in Schritt c) erhaltenen imprägnierten Vorläufers bei einer Temperatur von 80 °C bis 350 °C, um einen getrockneten, imprägnierten Vorläufer zu erhalten, und
e) Behandeln des in Schritt d) erhaltenen getrockneten, imprägnierten Vorläufers bei einer Temperatur von 350 °C bis 700 °C, um den geträgerten Metallkatalysator zu erhalten;
wobei das Gesamtporenvolumen des Katalysatorträgers durch Hg-Porosimetrie bestimmt wird; und
wobei das Reduktionsmittel eine Verbindung ist, die in der Lage ist, das Übergangsmetall aus dem in Schritt c) verwendeten zweiten flüssigen Medium zu reduzieren.

2. Zweimetallkatalysator, der durch das Verfahren nach Anspruch 1 erhalten wird, umfassend zwei Übergangsmetalle und einen Träger, wobei der Katalysator eine äußere Schalenregion, die eine maximalen Tiefe von 200 µm aufweist, und eine Mitte umfasst, wobei mindestens zu 90 % der Gesamtmenge die zwei Übergangsmetalle in der äußeren Schalenregion verteilt sind, und wobei mindestens zu 90 % der Gesamtmenge die in dem Katalysator enthaltenen Metallkristallite die zwei Übergangsmetalle umfassen; und wobei die zwei Übergangsmetalle aus der Gruppe ausgewählt sind, bestehend aus Pd, Pt, Cu, Ni, Au und Ag.

3. Katalysator nach Anspruch 2, umfassend Silber, Palladium und einen Träger, wobei der Katalysator eine äußere Schalenregion, die eine maximalen Tiefe von 200 µm aufweist, und eine Mitte umfasst, wobei mindestens zu 90 % der Gesamtmenge das Silber und das Palladium in der äußeren Schalenregion verteilt sind, und wobei mindestens zu 90 % der Gesamtmenge die in dem Katalysator enthaltenen Metallkristallite Silber und Palladium umfassen.

4. Katalysator nach Anspruch 3, wobei die Pd-Konzentration von 0,02 bis 0,05 Gew.-%, bezogen auf das Gesamtgewicht des Katalysators, beträgt.

5. Katalysator nach den Ansprüchen 3 oder 4, wobei das Gewichtsverhältnis des Palladiums zu dem Silber 1,7 bis 3,0 beträgt.

6. Verfahren für die Hydrierung, vorzugsweise für die selektive Hydrierung, einer Kohlenwasserstoffzuführung, die Acetylen enthält, vorzugsweise umfassend eine erste und eine zweite Gruppe ungesättigter Kohlenwasserstoffe, wobei die Kohlenwasserstoffzuführung unter geeigneten Hydrierungsbedingungen mit dem Katalysator nach einem der Ansprüche 2 bis 5 in Kontakt gebracht wird, wobei die ungesättigten Kohlenwasserstoffe in der Kohlenwasserstoffzuführung, vorzugsweise in der ersten Gruppe, hydriert werden.

7. Verfahren für die Hydrierung nach Anspruch 6, wobei die Kohlenwasserstoffzuführung in ihrer ersten Gruppe ungesättigter Kohlenwasserstoffe Acetylen umfasst.

8. Verfahren für die Hydrierung nach Anspruch 6 oder 7, wobei die Kohlenwasserstoffzuführung bei einer Temperatur von 10 bis 250 °C mit dem Katalysator in Kontakt gebracht wird.

9. Verfahren für die Hydrierung nach einem der Ansprüche 6 bis 8, wobei die Kohlenwasserstoffzuführung bei einem Druck von 0,5 bis 90 Bar mit dem Katalysator in Kontakt gebracht wird.

10. Verfahren für die Hydrierung nach einem der Ansprüche 6 bis 9, wobei die Kohlenwasserstoffzuführung mit dem Katalysator bei einer GHSV (stündliche Raumgeschwindigkeit) von 1000 bis 15000 v/vh in Kontakt gebracht wird.

11. Verfahren für die Hydrierung nach einem der Ansprüche 6 bis 10, wobei die Kohlenwasserstoffzuführung mit einem Wasserstoffstrom in Kontakt gebracht wird.

## Revendications

1. Procédé de production d'un catalyseur métallique supporté, comprenant les étapes suivantes :
a) la fourniture d'un support de catalyseur,
b) l'application d'un premier milieu liquide comprenant au moins un agent réducteur choisi dans le groupe constitué d'hydrazine, d'acide formique et de formaldéhyde sur le support de catalyseur fourni à l'étape a), 10 à 70 % du volume poreux total du support de catalyseur fourni dans l'étape a) étant rempli avec le premier milieu liquide,
c) immédiatement après l'étape b), l'imprégnation du support de catalyseur obtenu à l'étape b) avec un second milieu liquide comprenant deux métaux de transition choisis dans le groupe constitué de Pd, de Pt, de Cu, de Ni, d'Au et d'Ag afin d'obtenir un précurseur imprégné, au moins 91 % du volume poreux total du support de catalyseur fourni à l'étape a) étant rempli avec le premier et le second milieu liquide,
d) le séchage du précurseur imprégné obtenu à l'étape c) à une température allant de 80 °C à 350 °C afin d'obtenir un précurseur imprégné séché et
e) le traitement du précurseur séché et imprégné obtenu à l'étape d) à une température allant de 350 °C à 700 °C afin d'obtenir le catalyseur métallique supporté ;
le volume poreux total du support de catalyseur étant déterminé par porosimétrie Hg ; et
l'agent réducteur étant un composé qui est capable de réduire le métal de transition du second milieu liquide qui est utilisé à l'étape c).

2. Catalyseur à deux métaux obtenu par le procédé de la revendication 1, comprenant deux métaux de transition et un support, lequel catalyseur comprend une région d'enveloppe externe ayant une profondeur maximale de 200 um et un centre, au moins 90 % de la quantité totale des deux métaux de transition étant répartis dans la région d'enveloppe externe et au moins 90 % de la quantité totale des cristallites métalliques contenues dans le catalyseur comprenant les deux métaux de transition ; et les deux métaux de transition étant choisis dans le groupe constitué de Pd, de Pt, de Cu, de Ni, d'Au et d'Ag.

3. Catalyseur selon la revendication 2, comprenant de l'argent, du palladium et un support, lequel catalyseur comprend une région d'enveloppe externe ayant une profondeur maximale de 200 um et un centre, au moins 90 % de la quantité totale d'argent et de palladium étant répartis dans la région de l'enveloppe externe et au moins 90 % de la quantité totale de cristallites métalliques contenues dans le catalyseur comprenant de l'argent et du palladium.

4. Catalyseur selon la revendication 3, dans lequel la concentration en Pd est de 0,02 et 0,05 % en poids, en fonction du poids total du catalyseur.

5. Catalyseur selon les revendications 3 ou 4, dans lequel le rapport pondéral du palladium à l'argent est de 1,7 à 3,0.

6. Procédé d'hydrogénation, de préférence d'hydrogénation sélective, d'une charge d'hydrocarbures comprenant de l'acétylène, comprenant de préférence un premier et un second groupe d'hydrocarbures insaturés, dans lequel la charge d'hydrocarbures est mise en contact dans des conditions d'hydrogénation appropriées avec le catalyseur selon l'une quelconque des revendications 2 à 5, les hydrocarbures insaturés de la charge hydrocarbonée, de préférence du premier groupe, sont hydrogénés.

7. Procédé d'hydrogénation selon la revendication 6, dans lequel la charge d'hydrocarbures comprend dans son premier groupe d'hydrocarbures insaturés de l'acétylène.

8. Procédé d'hydrogénation selon la revendication 6 ou 7, dans lequel la charge d'hydrocarbures est mise en contact avec le catalyseur à une température allant de 10 à 250 °C.

9. Procédé d'hydrogénation selon l'une quelconque des revendications 6 à 8, dans lequel la charge d'hydrocarbures est mise en contact avec le catalyseur à une pression de 0,5 à 90 bars.

10. Procédé d'hydrogénation selon l'une quelconque des revendications 6 à 9, dans lequel la charge d'hydrocarbures est mise en contact avec le catalyseur à une GHSV (vitesse spatiale horaire du gaz) allant de 1 000 à 15 000 v/vh.

11. Procédé d'hydrogénation selon l'une quelconque des revendications 6 à 10, dans lequel la charge d'hydrocarbures est mise en contact avec un courant d'hydrogène.
